# EUROPÄISCHE PATENTANMELDUNG

(11) **EP 0 687 912 A2**
(43) Veröffentlichungstag der Anmeldung: **20.12.1995**
(21) Anmeldenummer: 95108930.9
(22) Anmeldetag: 09.06.1995
(51) Int. Cl.: G01N 35/10, B01L 3/02, C12Q 1/68

(54) **Verfahren zur mehrmaligen Entnahme von Flüssigkeiten, und analytische Bestimmung, die dieses Verfahren verwendet**

(30) Priorität: 15.06.1994 DE 4420900
(71) Anmelder: BOEHRINGER MANNHEIM GMBH, D-68298 Mannheim (DE)
(72) Erfinder: Bienhaus, Gerhard, Dr., D-82407 Wielenbach (DE); Lange, Hans, D-68623 Lampertheim (DE); Walter, Thomas, Dr., D-83673 Bad Tölz (DE)

(57) **Zusammenfassung**

Verfahren zur mehrmaligen Entnahme von Flüssigkeiten aus einem oder mehreren Vorratsbehältern in einen Entnahmebehälter, wobei zur Vermeidung von Kontaminationen Schutzspitzen auf eine Pipettenspitze aufgesetzt werden. Ein erfindungsgemäßes Analyseverfahren enthält die Schritte:
a) Aufnahme einer Probenflüssigkeit, in welcher der zu bestimmende Analyt enthalten ist durch eine Öffnung (11) in einen ersten Entnahmebehälter (10),
b) Immobilisierung des Analyten in dem ersten Entnahmebehälter (10),
c) Entfernung der Flüssigkeit aus dem Entnahmebehälter (10),
d) Befestigen einer Schutzspitze (20) mit einer Öffnung (21) an der ersten Öffnung (11) des Entnahmebehälters (10),
e) Aufnahme einer zweiten Flüssigkeit in den Entnahmebehälter (10) durch die Öffnungen (21,11).

## Beschreibung

Gegenstand der Erfindung ist ein Verfahren zur mehrmaligen Entnahme von Flüssigkeiten, ein Verfahren zur qualitativen oder quantitativen Bestimmung eines Analyten aus einer Flüssigkeit und eine in diesen Verfahren besonders vorteilhaft einsetzbare Vorrichtung.

In der klinischen und medizinischen Diagnostik sind Verfahren zur Analyse von Körperflüssigkeiten schon längere Zeit für die Diagnose von Krankheiten hilfreich. In neuerer Zeit wurde auch die Analyse von Nukleinsäuren für die Diagnose verwendet. Neben der in jüngerer Zeit möglichen Entschlüsselung des genetischen Codes vieler Organismen hat auch die Durchführung von Amplifikationsreaktionen keine weitere Verbreitung von nukleinsäureanalytischen Tests ermöglicht. Die Amplifikationsreaktionen ermöglichen auch den Nachweis kleinerer Mengen von Nukleinsäuren. Speziell für die Durchführung von Amplifikationen nach dem PCR-Prinzip (z. B. USA 4,683,195) ist eine besonders sorgfältige Probenbereitung zur Isolierung der Nukleinsäure erforderlich, da viele biologische Substanzen die in diesem Verfahren eingesetzte Polymerase stören. Wegen der äußerst hohen Sensitivität dieser Methode ist außerdem ein absolut verschleppungsfreies Arbeiten erforderlich.

Zur Anreicherung von Nukleinsäuren ist in EP-A-0 389 063 ein Verfahren beschrieben, bei dem die Bindung von Nukleinsäuren an Glaspartikel in Gegenwart von Guadiniumthiocyanathaltigen Puffern als besonders effizient hervorgehoben wird. Darüberhinaus beschreiben DE-A-41 39 664, DE-A-41 27 276 und WO 93/11221 die Verwendung von Glasvliesen als Trennmaterial, wobei wegen der Zentrifugationsschritte spezielle Vorrichtungen erforderlich sind. Die bisherigen Plastikteile, die die oben erwähnten Verfahren ausnutzen, gehorchen dem sogenannten "spin-column"-Verfahren, bei dem Zentrifugalkräfte benutzt werden, um die Lösungen durch die Glasvliese zu pressen. Dazu werden verschiedenste Teile als Einsätze für sogenannte Spin-column-Röhrchen verwandt.

In EP-A-0 588 564 sind Pipettenspitzen beschrieben, die an ihrem unteren, engeren Ende eine Membran aufweisen, an welche Analyten immobilisiert werden können. Diese Ausführungsform ist besonders anfällig für die Verschleppung von Flüssigkeiten von einem Gefäß in ein anderes, da Flüssigkeiten sich erfahrungsgemäß nicht vollständig aus solchen Membranen entfernen lassen und diese bei Inkontaktbringen der Pipettenspitze mit einer Flüssigkeit aus einem weiteren Behälter besonders leicht in diesen übertreten können.

Aufgabe der vorliegenden Erfindung ist das zur Verfügungstellen von Verfahren und Vorrichtungen, mit denen die Verschleppungen von Reagenzien von einer Bestimmung auf eine andere vermieden werden kann.

Gegenstand der Erfindung ist ein Verfahren zur mehrmaligen Entnahme von Flüssigkeiten aus einem oder mehreren Vorratsbehältern in einen Entnahmebehälter, gekennzeichnet durch die Schritte
a) erste Entnahme einer ersten Flüssigkeit in den Entnahmebehälter durch eine erste Öffnung des Entnahmebehälters,
b) Befestigung einer Schutzspitze über der ersten Öffnung des Entnahmebehälters, wobei die Schutzspitze eine Öffnung aufweist und
c) zweite Entnahme einer zweiten Flüssigkeit in den Entnahmebehälter durch die Öffnung des Entnahmebehälters und der Schutzspitze.

Ferner ist Gegenstand der Erfindung ein Verfahren zur qualitativen und quantitativen Bestimmung eines Analyten, welcher dieses Verfahren benutzt. Ein wesentlicher Gedanke bei der vorliegenden Erfindung ist es, den Übertritt von Probenflüssigkeit in Gefäße zu reduzieren, aus denen Reagenzlösung in die Probenflüssigkeit entnommen wird. Dadurch wird eine kontaminationsreduzierte Entnahme von Lösungen aus Reagenzflaschen möglich. Dieser Gedanke läßt sich auf beliebige Flüssigkeiten erweitern.

Verfahren zur mehrmaligen Entnahme von Flüssigkeiten aus einem oder mehreren Behältern sind dadurch charakterisiert, daß mehr als einmal Volumina einer Flüssigkeit aus demselben Behälter oder Volumina verschiedener Flüssigkeiten aus mehreren Behältern in einen Entnahmebehälter aufgenommen werden. Die Behälter, aus denen die Flüssigkeit entnommen wird, können beliebige Behälter, z. B. Vorratsbehälter, Tubes oder Flaschen sein. Probenvorratsbehälter sind Gefäße, die eine Probenflüssigkeit beinhalten, z. B. Primärcups oder Tubes. Reagenzvorratsbehälter enthalten Flüssigkeiten, die für die Durchführung einer chemischen Reaktion, z. B. einer Nachweisreaktion, benötigte Komponenten enthalten. Besonders bevorzugt enthalten diese Behälter Volumina zur Durchführung von mehr als 100 Reaktionen.

Als Probenflüssigkeit wird eine Flüssigkeit bezeichnet, die einen zu bestimmenden Analyten, z. B. eine Nukleinsäure, eine Zelle, ein Antigen, einen Antikörper oder ähnliches enthält. Geeignete Probenflüssigkeiten sind daher Körperflüssigkeiten wie Blut oder Urin, oder davon durch Zugabe weiterer Komponenten oder Entfernung gewisser Komponenten abgeleitete Flüssigkeiten, wie z. B. Serum oder Plasma.

Der Entnahmebehälter, in den die Flüssigkeiten aufgenommen werden, hat bevorzugt zwei Öffnungen, nämlich eine erste Öffnung, durch welche die Flüssigkeit in den Entnahmebehälter aufgenommen wird und eine zweite Öffnung, über die Gas oder Flüssigkeit aus dem Entnahmebehälter entnommen werden kann. Die erste Öffnung des Entnahmebehälters hat bevorzugt einen Querschnitt von 0.1 bis 5 mm² und ist bevorzugt kleiner als die zweite Öffnung. Die zweite Öffnung hat bevorzugt den Querschnitt von 3 bis 20 mm² und hat bevorzugt die Form eines definierten, standardisierten Aufnahmeflanschs. Aufgabe dieses Aufnahmeflanschs ist die Möglichkeit, den Entnahmebehälter an ein Gerät anzuschließen, mit dem die Flüssigkeitsentnahme bewirkt werden kann, z. B. einer manuellen Pipettierhilfe oder einem automatischen Pipettiergerät. Solche Geräte zum Bearbeiten von Flüssigkeitsvolumina sind dem Fachmann bekannt, z. B. die Tecan-Pipettierautomaten. In diesem Fall hat der Entnahmebehälter bevorzugt eine obere Öffnung, die an den Pipettierarm des Tecan-Gerätes angepaßt ist. Durch die Form der Öffnungen ergibt sich die bevorzugt im wesentlichen konische Form des Innern des Behälters und praktikablerweise auch des Äußeren des Entnahmebehälters. Die Wände des Entnahmebehälters bestehen bevorzugt aus gegenüber den zu entnehmenden Flüssigkeiten und gegebenenfalls mit ihnen durchgeführten Reaktionen inerten Kunststoff, z. B. Polypropylen, Polyäthylen, Polycarbonat, Polyurethan. Der durch die Behälterinnenwand gebildete Innenraum ist bevorzugt selbst nicht kapillar. Der Entnahmebehälter kann jedoch in seinem Innenraum Mittel enthalten, die für die Durchführung einer Reaktion benötigt werden. Dies können beispielsweise chemische Reagenzien, jedoch auch Materialien zur Immobilisierung einzelner Komponenten der ersten Flüssigkeit, insbesondere nachzuweisende Bestandteile der Flüssigkeit enthalten. Als Materialien zur Immobilisierung haben sich z. B. Gewebe aus Polyester, Polyamid, Polycarbonat, Zellulose, Nitrozellulose oder Glas bewährt. Diese Materialien können jedoch auch andere äußere Formen haben, z. B. Perlen (Beads) oder Vliese. Für den Fall einer Immobilisierung von Nukleinsäuren an diesen Materialien ist die Verwendung von Glasvliesen bevorzugt. Für andere Zwecke können beispielsweise auch die in der Affinitätschromatographie üblichen Materialien verwendet werden.

Der Entnahmebehälter kann ferner Einbauten enthalten, wie sie zur Fixierung der Reagenzien bzw. Immobilisierungsmaterialien erforderlich sind. Im Falle der Immobilisierung von Nukleinsäuren an einem Glasvlies ist beispielsweise das Glasvlies in Richtung auf die beiden Öffnungen des Entnahmebehälters bevorzugt mit einem inerten Plastiknetz festgehalten. Die im Entnahmebehälter vorhandenen Mittel sind bevorzugt so weit von der ersten Öffnung entfernt angebracht, daß sie nicht schon bei Inkontaktbringen der Öffnung mit den Flüssigkeiten in Kontakt kommen. Insbesondere sind die Mittel bevorzugt nicht von außen auf die Öffnung des Entnahmebehälters aufgebracht.

In einem bevorzugten Fall hat der Entnahmebehälter die Form einer üblichen Pipettenspitze. Dadurch ist die Adaptierbarkeit des erfindungsgemäßen Verfahrens für Pipettierautomaten gegeben. Der Entnahmebehälter weist jedoch auch einen Bereich auf, an dem eine Schutzspitze befestigt werden kann. Dies kann in analoger Weise zur Befestigung des Entnahmebehälters an dem Pipettierarm geschehen. Dafür weist auch das Äußere des Entnahmebehälters eine konische Form auf, die zum Aufnahmeflansch der Schutzspitze paßt. Die Haftung der Schutzspitze auf dem Entnahmebehälter kann dadurch noch verbessert werden, daß eine Möglichkeit zum Einrasten vorgesehen ist.

Ein wesentlicher Bestandteil der vorliegenden Erfindung ist die Verwendung einer Schutzspitze, die verhindert, daß die äußere Oberfläche des Entnahmebehälters direkt mit der zweiten Flüssigkeit in Kontakt kommt. Dies ist insofern wichtig, als an der äußeren Oberfläche des Entnahmebehälters sich gelegentlich noch Reste der ersten Flüssigkeit befinden können, die dann in die zweite Flüssigkeit übertreten und für eine Kontamination sorgen. Die Schutzspitze hat wie der Entnahmebehälter bevorzugt zwei Öffnungen, von denen die erste zur Entnahme der zweiten Flüssigkeit aus einem Vorratsbehälter dient. Die zweite Öffnung stellt den Anschluß an den Entnahmebehälter her. Bevorzugt ragt ein Teil des Entnahmebehälters, der die erste Öffnung beinhaltet, in die zweite Öffnung der Schutzspitze hinein. Die Schutzspitze sollte bevorzugt möglichst dicht mit der Außenwand des Entnahmebehälters abschließen, um Druck- oder Flüssigkeitsverluste zu vermeiden und weiterer Kontaminationsgefahr zu begegnen. Die Schutzspitze weist daher an ihrem Ende eine an die äußere Form des Entnahmebehälters im Bereich der ersten Öffnung angepaßte definierten, standardisierte Form auf. Als Material für die Schutzspitze kommt insbesondere Material in Frage, wie es auch für den Entnahmebehälter verwendet wird. Bevorzugt handelt es sich bei der Schutzspitze um ein billiges Wegwerfprodukt aus Kunststoff. Die Schutzspitze kann daher eine ähnliche Form wie die üblicherweise verwendeten Pipettenspitzen aufweisen. Ihr Innenraum kann jedoch sehr viel kleiner sein als die üblicherweise verwendeten Pipettenspitzen oder des Entnahmebehälters, da das aufgenommene Flüssigkeitsvolumen nicht darin Platz finden muß. Die Schutzspitze kann in ihrem Innenraum wie der Entnahmebehälter Mittel zur Durchführung einer Reaktion enthalten. Bevorzugt sind jedoch Pipettenspitzen, die keine solche Mittel enthalten.

Ein typischer Fall der Anwendung eines Verfahrens, bei dem eine mehrmalige Entnahme von Flüssigkeiten aus Vorratsbehältern stattfindet, sind Verfahren zur Bestimmung von Analyten aus einer Probenflüssigkeit. Gerade hier erweisen sich Kontaminationen als besonders schwerwiegend, da sie die Analysenwerte verfälschen können. Ebenfalls Gegenstand der Erfindung ist daher ein Verfahren zur qualitativen oder quantitativen Bestimmung eines Analyten enthaltend die Schritte
a) Aufnahme einer Probenflüssigkeit, in welcher der zu bestimmende Analyt enthalten ist durch eine Öffnung (11) in einen ersten Entnahmebehälter (10),
b) Immobilisierung des Analyten in dem ersten Entnahmebehälter (10),
c) Entfernung der Flüssigkeit aus dem Entnahmebehälter (10),
d) Befestigen einer Schutzspitze (20) mit einer Öffnung (21) an der ersten Öffnung (11) des Entnahmebehälters (10),
e) Aufnahme einer zweiten Flüssigkeit in den Entnahmebehälter (10) durch die Öffnungen (21, 11).

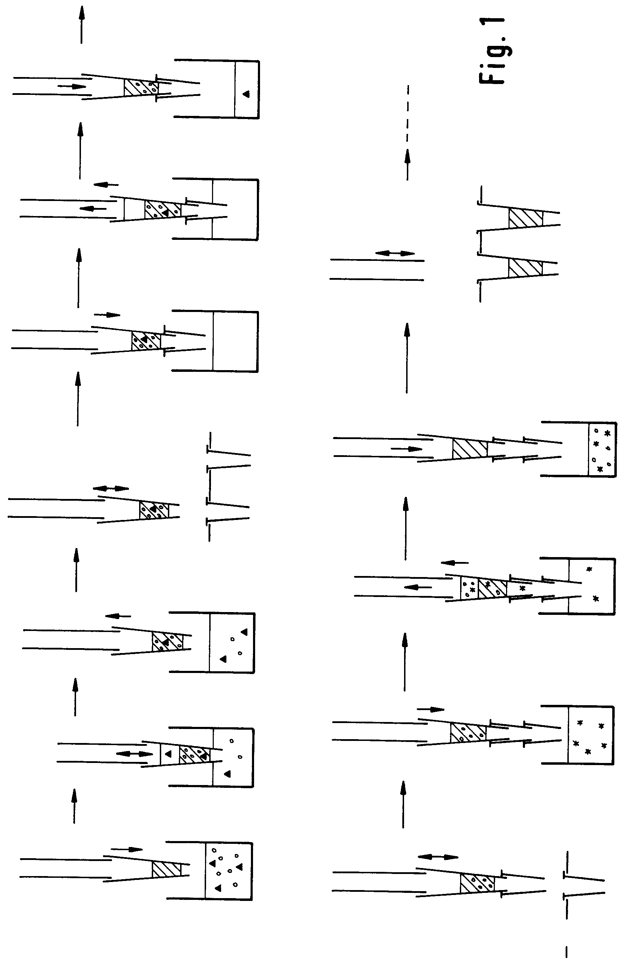

Im folgenden wird ein solches Isolierungsverfahren am Beispiel einer Nukleinsäureisolierung beschrieben. Hierbei wird auf Figur 1 Bezug genommen. In einem ersten Schritt wird ein bestimmtes Volumen einer Probenflüssigkeit auf beliebige Weise in den Entnahmebehälter überführt. Dies kann beispielsweise durch Anlegen eines Unterdrucks an der ersten Öffnung des Entnahmebehälters über die zweite Öffnung des Entnahmebehälters geschehen (z. B. Ausaugen mittels einer Pipettierhilfe oder eines Pipettierautomaten, wie z. B. der Firmen Tecan, Hamilton oder Beckmann). Dabei kann die Flüssigkeit auch mehrmals angesaugt und wieder ausgestoßen werden. Die Öffnung (11) des Entnahmebehälters ragt dabei in die Probenflüssigkeit im Vorratsbehälter ein. Der Entnahmebehälter enthält in seinem Innenraum ein durch inerte Plastiknetze über den gesamten Querschnitt des Entnahmebehälters eingegossenes Glasvlies. Die Probenflüssigkeit enthält die zu isolierende Nukleinsäure, bevorzugt in einem Puffer, der die Immobilisierung der Nukleinsäuren an Glasvliesen erleichtert, besonders bevorzugt GuSCN. Als Glasvlies wird ein Vlies WF 264, WF 265 (Whatman) verwendet. Nach einer Inkubationszeit von 1 sec.-30 min. bevorzugt 2-10 sec. wird die Flüssigkeit aus dem Entnahmebehälter in den Probenvorratsbehälter oder einen Abfallbehälter ausgestoßen. Die Nukleinsäuren verbleiben immobilisiert in dem Entnahmebehälter.

Auf die untere Spitze des Entnahmebehälters wird danach eine Schutzspitze aufgesteckt. Die Schutzspitzen können in einem Vortatsbehälter, z. B. auf dem Pipettierautomaten, vorrätig gehalten werden und durch Ansteuern einer Aufnahmeposition automatisch aufgenommen werden. Damit wird der untere Teil des Entnahmebehälters, der mit der zweiten Flüssigkeit in Kontakt kommen könnte, geschützt.

Anschließend kann der Entnahmebehälter in eine Position überführt werden, in der die Öffnung der Schutzspitze in eine Waschflüssigkeit hineinragt. Durch Ansaugen der Waschflüssigkeit aus dem Flüssigkeitsvorratsbehälter werden die immobilisierten Nukleinsäuren von etwa noch anhaltenden Probenflüssigkeitsresten befreit, die in die im Entnahmebehälter befindliche Flüssigkeit übertreten. Anschließend wird die Waschflüssigkeit zusammen mit den Verunreinigungen in einen Abfallbehälter ausgestoßen. Somit befinden sich die Nukleinsäuren in relativ reiner Form im Entnahmebehälter immobilisiert.

Anschließend wird eine weitere Schutzspitze aufgesteckt, so daß der untere Teil der ersten Schutzspitze durch die zweite Schutzspitze geschützt wird, so daß eventuell an der ersten Schutzspitze anhaftende Flüssigkeit nicht mit der dritten Flüssigkeit in Kontakt treten kann.

In einem weiteren Schritt wird der Entnahmebehälter so in eine Flüssigkeit eingetaucht, daß die Öffnung der zweiten Schutzspitze in die Flüssigkeit hineinragt. Die dritte Flüssigkeit ist eine Flüssigkeit, die eine Desimmobilisierung der Nukleinsäure von dem Glasvlies bewirkt. Insbesondere handelt es sich hier um eine Flüssigkeit mit Niedrigsalzpuffer. Sobald eine ausreichende Menge der Flüssigkeit aufgenommen wurde, so daß sich die Nukleinsäuren darin lösen können, wird nach einer eventuellen Wartezeit die Flüssigkeit zusammen mit den eluierten Nukleinsäuren in ein frisches Gefäß ausgestoßen. In diesem Gefäß kann eine Weiterbehandlung der Nukleinsäuren stattfinden, z. B. eine qualitative oder quantitative Bestimmung der Nukleinsäuren vorgenommen werden. Dazu kann das Gefäß bereits Reagenzien in fester oder flüssiger Form beinhalten oder diese werden erst später zugegeben.

Zum Abschluß des Verfahrens kann der Entnahmebehälter zusammen mit den Schutzspitzen vom Pipettierarm abgestreift und verworfen werden. Dies erledigt bevorzugt der Pipettierautomat. Für eine neue Bestimmung wird ein neuer Entnahmebehälter aufgesteckt.

Ein großer Vorteil des erfindungsgemäßen Verfahrens besteht darin, daß auf die beschriebene Weise unterschiedliche Tests miteinander kombiniert werden können, je nach dem ob ein erfindungsgemäßer Entnahmebehälter oder einfache Pipettenspitzen auf den Pipettierarm aufgesteckt werden.

Während in dem eben beschriebenen Verfahren die Flüssigkeit wieder aus der unteren Öffnung des Entnahmebehälters und der Schutzspitzen entfernt wird, ist es jedoch auch denkbar, daß die Flüssigkeiten den Entnahmebehälter durch die zweite Öffnung verlassen. Dies gilt insbesondere für die Schritte zur Immobilisierung der Nukleinsäuren an der festen Phase und die Waschschritte. Jedoch ist es auch möglich, die eluierten Nukleinsäuren anzusaugen und beispielsweise in einem Durchflußmeßgerät zu detektieren.

Eine Schutzspitze kann erfindungsgemäß immer darin aufgesteckt werden, wenn eine Kontamination vom Entnahmebehälter oder den darauf aufgesteckten Schutzspitzen in den (neuen) Flüssigkeitsbehälter (sowohl bei Entnahme einer weiteren Menge von Flüssigkeit aus dem schon einmal verwendeten Flüssigkeitsbehälter oder bei Entnahme einer Menge an (neuer) Flüssigkeit aus einem weiteren Flüssigkeitsbehälter) zu befürchten ist. So kann z. B. verhindert werden, daß Teile von gebrauchter Waschlösung, die evtl. noch dem Entnahmebehälter oder der Schutzspitze anhaften, in die frische Waschlösung übertreten und diese kontaminieren, was eine Verfälschung der danach unter Verwendung der gleichen Waschlösung durchgeführten Analysen zur Folge haben könnte.

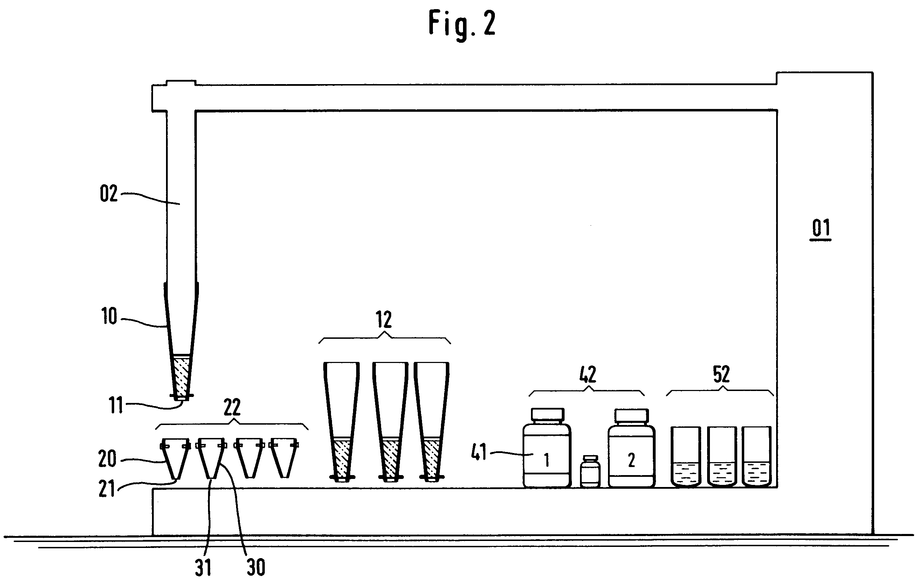

In Figur 2 ist schematisch ein Pipettierautomat (01) mit einem Pipettierarm (02), Entnahmebehältern (10) und (teilweise) in einem Magazin (12), Schutzspitzen (20) in einem Magazin (22), Reagenzien (41) (z. B. Waschflüssigkeiten und Elutionsflüssigkeiten in einem Reagenzmagazin (42) sowie Probenflüssigkeiten in Probenvorratsbehältern (52) gezeigt.

### Bezugszeichenliste

- 01: Pipettierautomat
- 02: Pipettierarm
- 10: Entnahmebehälter
- 11: Öffnung in 10
- 12: Magazin für 10
- 20: Schutzspitze
- 21: Öffnung in 20
- 22: Magazin für 20
- 30: zweite Schutzspitze
- 31: Öffnung in 30
- 41: Reagenzien
- 42: Magazin für 41
- 52: Probenvorratsbehälter

## Patentansprüche

1. Verfahren zur mehrmaligen Entnahme von Flüssigkeiten aus einem oder mehreren Vorratsbehältern in einen Entnahmebehälter gekennzeichnet durch die Schritte
a) erste Entnahme einer ersten Flüssigkeit in den Entnahmebehälter (10) durch eine erste Öffnung (11) des Entnahmebehälters
b) Befestigung einer Schutzspitze (20) über der ersten Öffnung (11) des Entnahmebehälters (10) wobei die Schutzspitze eine Öffnung (21) aufweist und
c) zweite Entnahme einer zweiten Flüssigkeit in den Entnahmebehälter (10) durch die Öffnungen (21, 11).

2. Verfahren gemäß Anspruch 1, dadurch gekennzeichnet, daß der Entnahmebehälter die Form einer Pipettenspitze hat.

3. Verfahren gemäß Anspruch 1, dadurch gekennzeichnet, daß der Entnahmebehälter Mittel zur Immobilisierung von Komponenten der ersten Flüssigkeit beinhaltet.

4. Verfahren zur Isolierung einer Komponente einer Probenflüssigkeit enthaltend die Schritte
a) Aufnahme einer Probenflüssigkeit, in welcher die Komponente enthalten ist, durch eine Öffnung (11) in einen Entnahmebehälter (10),
b) Immobilisierung der Komponente in dem Entnahmebehälter (10),
c) Entfernung der Flüssigkeit aus dem Entnahmebehälter,
d) Befestigung einer Schutzspitze (20) mit einer Öffnung (21) an der ersten Öffnung des Entnahmebehälters (10),
e) Aufnahme einer zweiten Flüssigkeit in den Entnahmebehälter (10) durch die Öffnungen (21, 11).

5. Verfahren gemäß Anspruch 4, dadurch gekennzeichnet, daß die zweite Flüssigkeit eine Lösung zum Waschen der immobilisierten Komponente ist.

6. Verfahren gemäß Anspruch 4, gekennzeichnet durch die weiteren Schritte
f) Befestigung einer zweiten Schutzspitze (30) mit einer Öffnung (31) an der ersten Schutzspitze,
g) Aufnahme einer dritten Flüssigkeit durch die Öffnungen (31, 21 und 11).

7. Verfahren gemäß Anspruch 4, dadurch gekennzeichnet, daß die dritte Flüssigkeit eine Lösung zur Aufhebung der Immobilisierung der Komponente ist.

8. Verfahren nach einem der Ansprüche 4 bis 7, dadurch gekennzeichnet, daß die Komponente eine Nukleinsäure ist.

9. Verfahren nach einem der Ansprüche 4 bis 7, dadurch gekennzeichnet, daß der Entnahmebehälter eine Plastikpipettenspitze ist.

10. Verfahren nach einem der Ansprüche 4 bis 7, dadurch gekennzeichnet, daß die Schutzspitze eine Plastikpipettenspitze ist.

11. Verfahren nach einem der Ansprüche 4 bis 10, dadurch gekennzeichnet, daß die Schutzspitze (20) Möglichkeiten zum Einrasten am Entnahmebehälter (10) aufweist.

12. Verfahren gemäß Anspruch 4, dadurch gekennzeichnet, daß der Entnahmebehälter (10) ein Glasvlies beinhaltet.

13. Verfahren zur qualitativen oder quantitativen Bestimmung eines Analyten enthaltend die Schritte
a) Aufnahme einer Probenflüssigkeit, in welcher der Analyt enthalten ist, durch eine Öffnung (11) in einen Entnahmebehälter (10),
b) Immobilisierung des Analyten in dem Entnahmebehälter (10),
c) Entfernung der Flüssigkeit aus dem Entnahmebehälter,
d) Befestigen einer Schutzspitze (20) mit einer Öffnung (21) an der ersten Öffnung (11) des Entnahmebehälters (10),
e) Aufnahme einer Elutionsflüssigkeit in den Entnahmebehälter (10) durch die Öffnungen (21, 11) und
f) Abgabe der den Analyten enthaltenden Elutionsflüssigkeit aus dem Entnahmebehälter.
